(19) Europäisches Patentamt
European Patent Office
Office européen des brevets

(11) Veröffentlichungsnummer: **0 441 922 B1**

(12) **EUROPÄISCHE PATENTSCHRIFT**

(45) Veröffentlichungstag der Patentschrift: **03.08.94**

(51) Int. Cl.[5]: **C07C 25/18**, C09K 19/30, C09K 19/12, C07C 43/225

(21) Anmeldenummer: **90912091.7**

(22) Anmeldetag: **24.08.90**

(86) Internationale Anmeldenummer:
**PCT/EP90/01411**

(87) Internationale Veröffentlichungsnummer:
**WO 91/03447 (21.03.91 91/07)**

# (54) DIFLUORBENZONITRILE UND FLÜSSIGKRISTALLINES MEDIUM.

(30) Priorität: **05.09.89 DE 3929418**
**21.12.89 DE 3942261**
**16.01.90 DE 4001022**

(43) Veröffentlichungstag der Anmeldung:
**21.08.91 Patentblatt 91/34**

(45) Bekanntmachung des Hinweises auf die Patenterteilung:
**03.08.94 Patentblatt 94/31**

(84) Benannte Vertragsstaaten:
**CH DE FR GB IT LI NL**

(56) Entgegenhaltungen:
**EP-A- 0 316 715**
**EP-A- 0 317 175**
**WO-A-89/08102**

(73) Patentinhaber: **MERCK PATENT GmbH**
**Postfach,**
**Frankfurter Strasse 250**
**D-64271 Darmstadt(DE)**

(72) Erfinder: **REIFFENRATH, Volker**
**Jahnstra e 18**
**D-6101 Ro dorf(DE)**
Erfinder: **KURMEIER, Hans-Adolf**
**Hinter der Schule 3a**
**D-6104 Seeheim-Jugenheim(DE)**
Erfinder: **SCHEUBLE, Bernhard**
**Grundweg 3**
**D-6104 Seeheim-Jugenheim(DE)**
Erfinder: **COATES, David 87 Sopwith Crescent**
**Merley**
**Wimborne**
**Dorset BH21 3SW(GB)**
Erfinder: **SMITH, Graham**
**10 Henbury Close**
**Canford Heath**
**Poole Dorset BH17 8AX(GB)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein flüssigkristallines Medium für elektrooptische Anzeigeelemente mit mindestens zwei flüssigkristallinen Komponenten, dadurch gekennzeichnet, daß mindestens eine Kompenente ein Difluorbenzonitril der Formel I ist

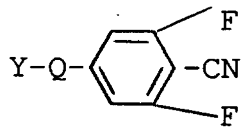 I

worin
Y Alkyl, Alkenyl, Alkoxy, Oxaalkyl oder Alkenyloxy mit jeweils 1 bis 12 C-Atomen und
Q einen Rest ausgewählt aus der Gruppe bestehend aus

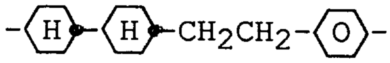, 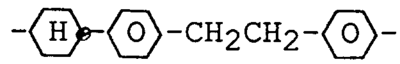,
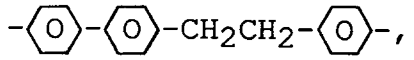, 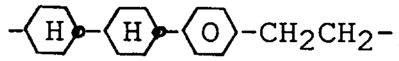,
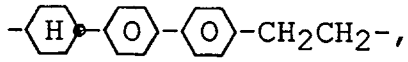, 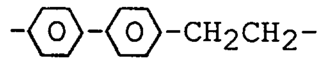,
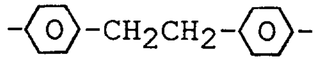, 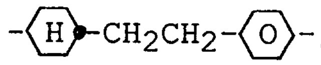,
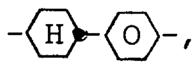, 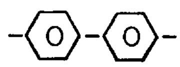 und 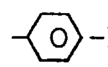 bedeutet.

Die Erfindung betrifft weiterhin elektrooptische Anzeigeelemente, die die erfindungsgemäßen flüssigkristallinen Medien enthalten. Die Erfindung betrifft weiterhin Difluorbenzonitrile der Formel I mit der Maßgabe, daß Q nicht 1,4-Phenylen ist.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine sehr hohe dielektrische Anisotropie bei gleichzeitig gutem Tieftemperaturverhalten

Es wurde nun gefunden, daß Verbindungen der Formel I als Komponenten flüssigkristalliner Medien vorzüglich geeignet sind. Insbesondere verfügen sie über vergleichsweise niedere Viskositäten. Mit ihrer Hilfe lassen sich stabile flüssigkristalline Medien mit breitem Mesophasenbereich und vorteilhaften Werten für die optische und dielektrische Anisotropie erhalten.

Flüssigkristalle der Formel

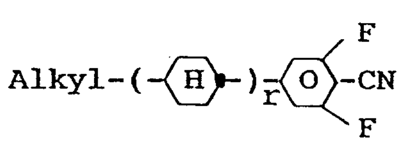

r = 1 oder 2
sind bereits aus DE 3209178 bekannt. Aus der JP 62-103057 sind Verbindungen der Formeln

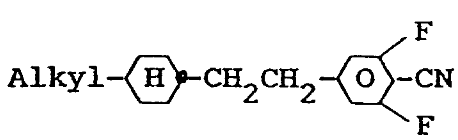

und

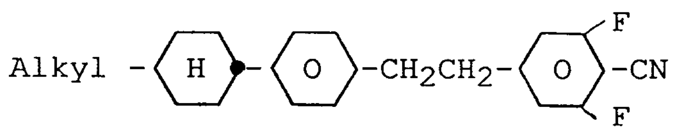

bekannt. In JP 63-216858 und USP 4,853,152 schließlich werden Verbindungen der Formeln

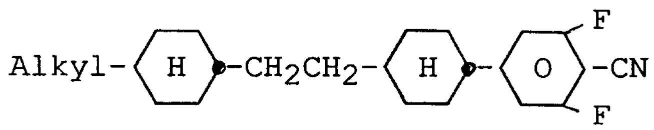

und

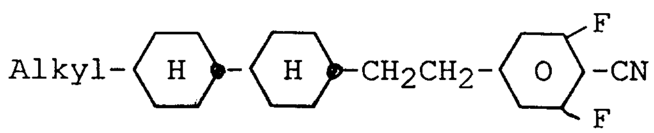

beschrieben. In WO 89/08,102 wird

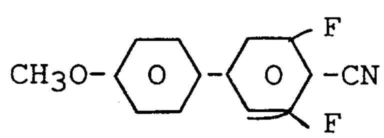

als Zwischenprodukt zur Herstellung von Esterverbindungen mit einer terminalen 3,5-Difluor-4-cyanophenylgruppe genannt.

Im Hinblick auf die verschiedensten Einsatzbereiche derartiger Verbindungen mit sehr hohem $\Delta\epsilon$ war es jedoch wünschenswert, weitere Verbindungen zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil

Gegenstand der Erfindung sind somit die Verbindungen der Formel I mit der Maßgabe, daß Q nicht 1,4-Phenylen ist, sowie die Verwendung von Verbindungen der Formel I als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektrooptische Anzeigeelemente, die derartige Medien enthalten.

Die Verbindungen der Formel I umfassen dementsprechend Verbindungen der Teilformeln Ia bis Il:

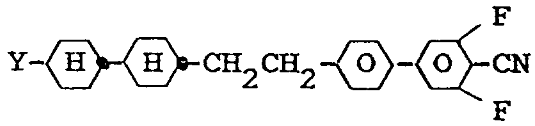

Ia

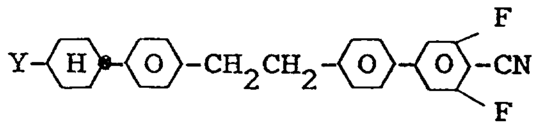

Ib

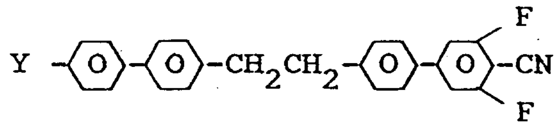

Ic

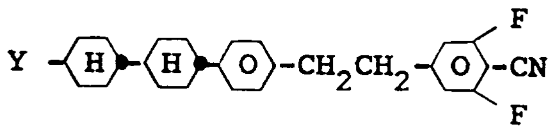

Id

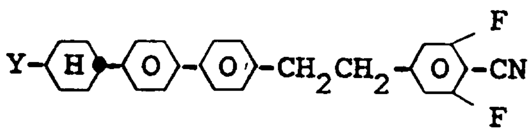

Ie

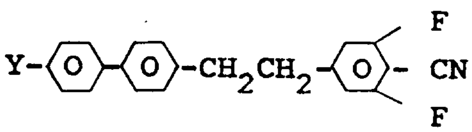

If

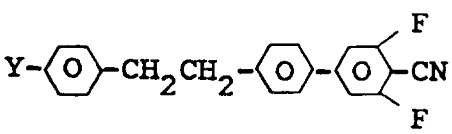

Ig

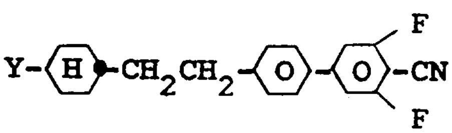

Ih

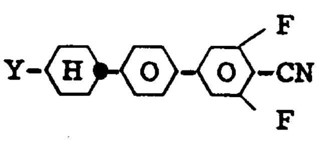

Ii

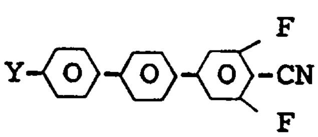

Ik

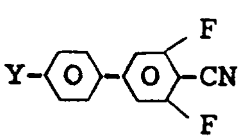

Il

Darunter sind diejenigen der Formeln Ia, Ib, Id, If, Ig, Ih und Ii besonders bevorzugt.

Y bedeutet vorzugsweise Alkyl, ferner Alkoxy.

Falls Y einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8-

oder 9-Oxadecyl.

Falls Y einen Alkylrest bedeutet, in dem eine $CH_2$-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Verbindungen der Formeln I mit verzweigten Flügelgruppen Y können gelegentlich wegen einer besseren Löslichkeit in den üblichen flüssigkristallinen Basismaterialien von Bedeutung sein, insbesondere aber als chirale Dotierstoffe, wenn sie optisch aktiv sind.

Verbindungen der Formel I mit $S_A$-Phasen eignen sich beispielsweise für thermisch adressierte Displays.

Verzweigte Gruppen dieser Art enthalten in der Regel nicht mehr als eine Kettenverzweigung. Bevorzugte verzweigte Reste Y sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die erfindungsgemäßen Verbindungen können z.B. hergestellt, indem man eine Verbindung der Formel II,

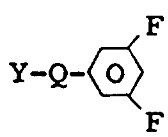

II

worin Y und Q die angegebene Bedeutung haben, gemäß folgendem Reaktionsschema metalliert und anschließend mit einem geeigneten Elektrophil umsetzt:

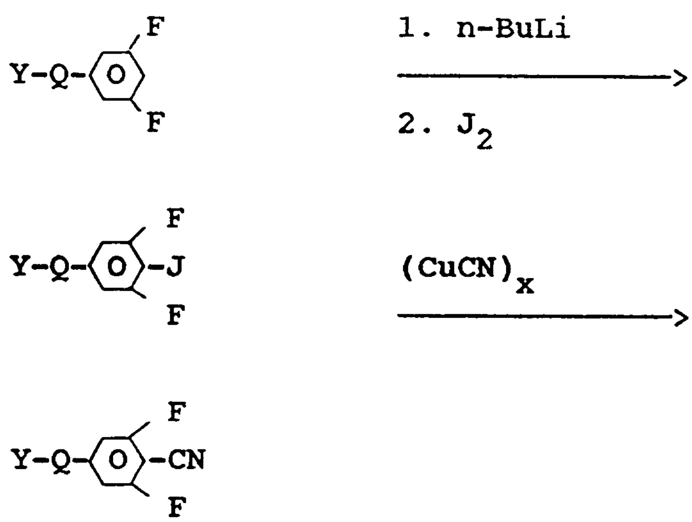

Weitere Synthesemethoden sind für den Fachmann augenscheinlich. Beispielsweise können in 5-Position entsprechend substituierte 1,3-Difluorbenzol-Verbindungen gemäß obigem Schema in die 2-Cyan-1,3-difluor-Verbindungen überführt werden und der Rest Y-Q- anschließend durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) angeführt werden.

Die Verbindungen der Formel II können beispielsweise nach folgenden Syntheseschemata hergestellt werden:

Schema 1

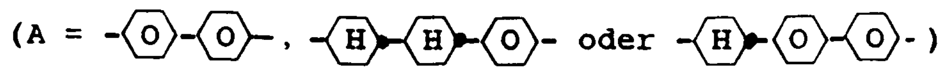

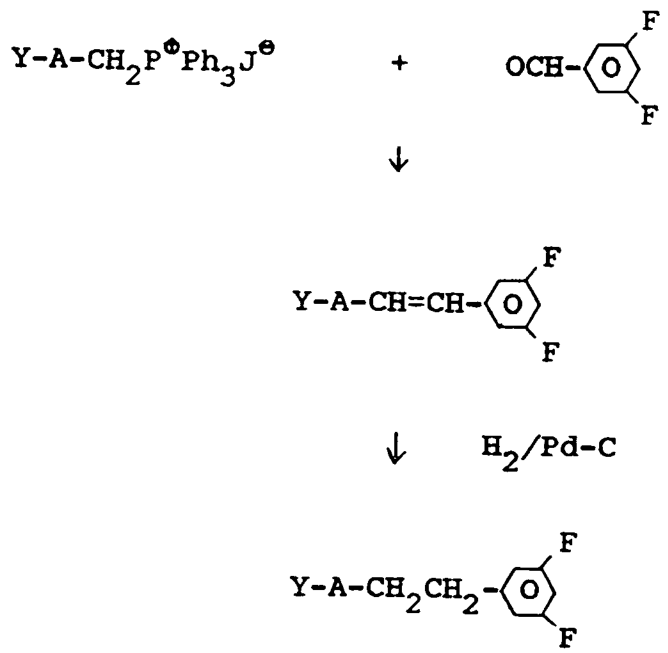


Schema 2

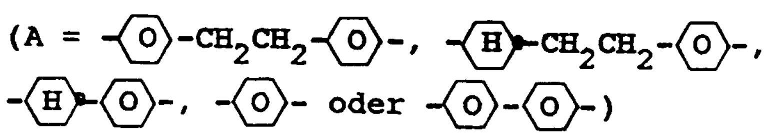

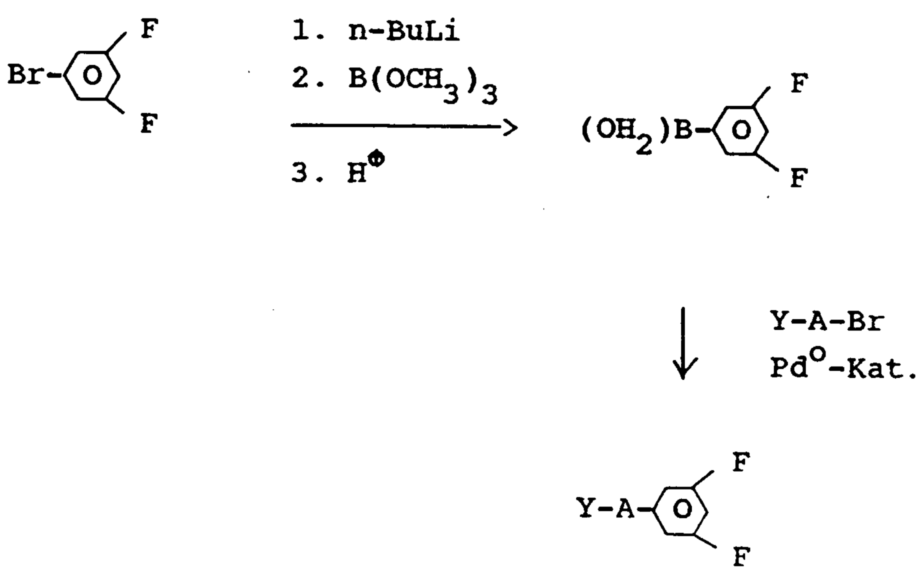

<u>Schema 3</u> (A = 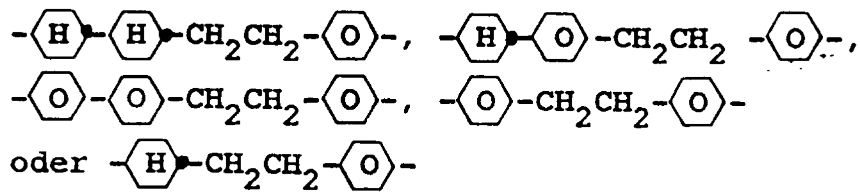


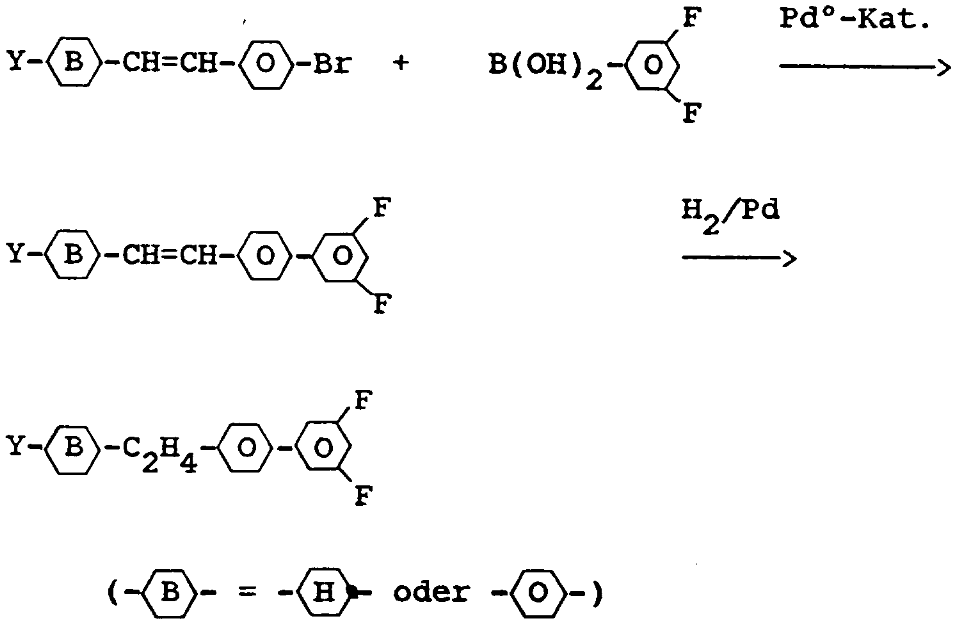


Ein besonders bevorzugtes Verfahren für die bevorzugten Verbindungen der Teilformeln Ia, Ib und Ic ist in folgendem Schema angegeben:

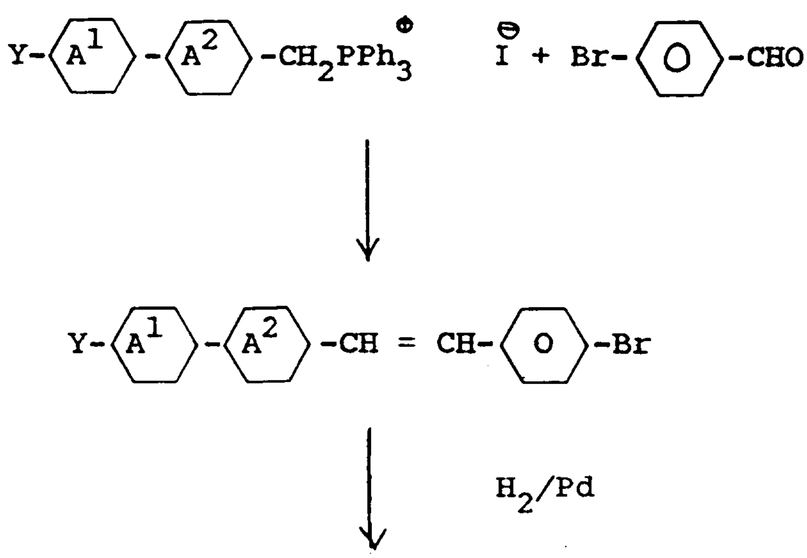

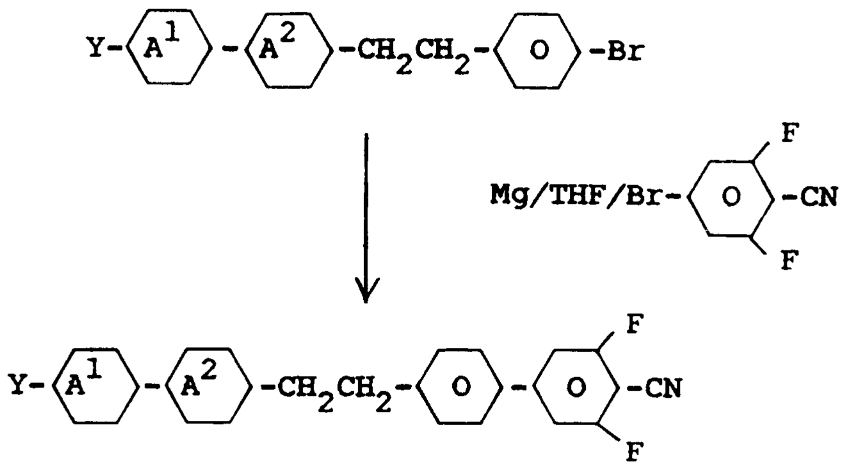


($A^1$ und $A^2$ jeweils -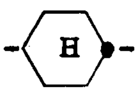- oder -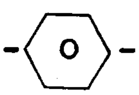-)

Die Ausgangsmaterialien sind entweder bekannt oder können in Analogie zu bekannten Verbindungen hergestellt werden.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cycloheyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-$CH_2CH_2$-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R'' voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R'' -F, -Cl, -NCS oder $-(O)_iCH_{3-(k+l)}F_kCl_l$, wobei i 0 oder 1 und k + l 1, 2 oder 3 sind; die Verbindungen, in denen R'' diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R'' die Bedeutung -F, -Cl, -NCS, $-CF_3$, $-OCHF_2$ oder $-OCF_3$ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R'' -CN; diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise

Gruppe A: 0 bis 90 %, vorzugsweise 20 bis 90 %, insbesondere 30 bis 90 %
Gruppe B: 0 bis 80 %, vorzugsweise 10 bis 80 %, insbesondere 10 bis 65 %
Gruppe C: 0 bis 80 %, vorzugsweise 5 bis 80 %, insbesondere 5 bis 50 %

wobei die Summe der Massenanteile der in den jeweiligen erfindungsgemäßen Medien enthaltenen Verbindungen aus den Gruppen A und/oder B und/oder C vorzugsweise 5 %-90 % und insbesondere 10 % bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. Vor- und nachstehend bedeuten Potenzangaben Gewichtsprozent. Alle Temperaturen sind in Grad Celsius angegeben. Fp. bedeutet Schmelzpunkt Kp = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. $\Delta n$ bedeutet optische Anisotropie (589 nm, 20 °C) und die Viskosität

(mm$^2$/sec) wurde bei 20 °C bestimmt.

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:

| | |
|---|---|
| DAST | Diethylaminoschwefeltrifluorid |
| DCC | Dicyclohexylcarbodiimid |
| DDQ | Dichlordicyanobenzochinon |
| DIBALH | Diisobutylaluminiumhydrid |
| KOT | Kalium-tertiär-butanolat |
| THF | Tetrahydrofuran |
| pTSOH | p-Toluolsulfonsäure |
| TMEDA | Tetramethylethylendiamin |

Beispiel 1

Eine Lösung von 0,1 m 1-(4'-n-Pentylbiphenyl-4-yl)-2-(3,5-difluorphenyl)-ethan (Schmelzpunkt 59°, hergestellt nach Schema 1) und 0,1 m TMEDA in 300 ml THF wird bei ca. -90° mit 0,1 m n-BuLi (1,5 M in Hexan) tropfenweise versetzt. Man rührt noch 30 min. bei dieser Temperatur und setzt dann eine Lsg. von 0,1 m $J_2$ in 70 ml THF langsam zu. Nach beendeter Zugabe läßt man auf -20° erwärmen und hydrolysiert mit $H_2O$. Durch Zugabe von Diäthylether wird das Produkt vollständig in Lösung gebracht und überschüssiges $J_2$ durch Waschen mit Natriumthiosulfatlsg. und $H_2O$ entfernt. Nach dem Eindampfen verbleibt das Produkt als Rückstand und wird ohne weitere Aufreinigung mit 0,12 m $(CuCN)_x$ und 100 ml NMP 4 h auf 170 °C im Ölbad erwärmt. Nach dieser Zeit wird das abgekühlte Reaktionsgemisch mit Wasser und $CH_2CH_2$ versetzt, die org. Phase gewaschen, getrocknet und eingedampft. Das reine Produkt läßt sich durch Chromatographie und Kristallisation gewinnen. Man erhält 1-(4'-n-Pentylbiphenyl-4-yl)-2-(4-cyan-3,5-difluor-phenyl)-ethan.

Beispiel 2 bis 39:

Analog erhält man aus den entsprechenden Vorstufen der Formel II die folgenden erfindungsgemäßen Verbindungen:

| | Y | Q |
|---|---|---|
| (2) | Ethyl | 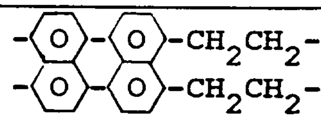 |
| (3) | Methoxy | |
| (8) | Ethoxy | 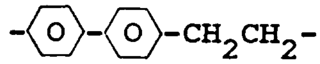 |
| (9) | n-Butyl | 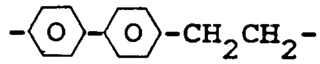 |
| (10) | n-Pentyl | 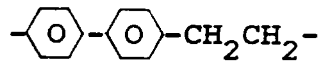 |
| (11) | Methoxymethyl | 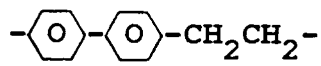 |
| (12) | n-Propyl | 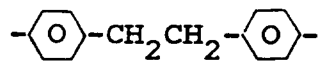 |
| (13) | n-Pentyl | 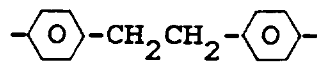 |
| (14) | n-Propyl | 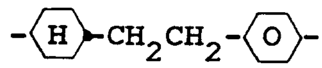 |
| (15) | n-Pentyl | 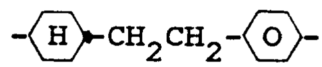 |
| (16) | n-Propyl | 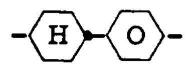 |
| (17) | n-Butyl | 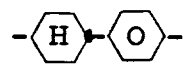 |

| | Y | Q |
|---|---|---|
| (18) | n-Pentyl | 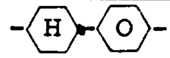 |
| (19) | n-Propyl | 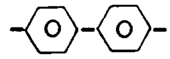 |
| (20) | n-Pentyl | 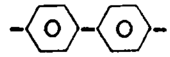, K 107 N 121.2; $\Delta\varepsilon$ = 34,5; $\Delta n$ = 0,283 |
| (21) | n-Propyl | 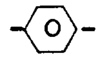 |
| (22) | n-Butyl | 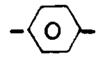 |
| (19) | n-Pentyl | 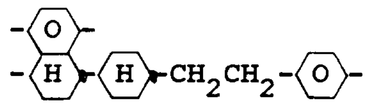 |
| (20) | Ethyl | |
| (21) | n-Propyl | 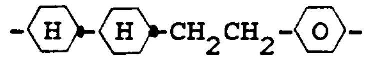 |
| (22) | n-Butyl | 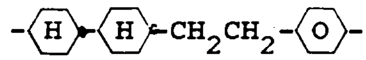 |
| (23) | n-Pentyl | 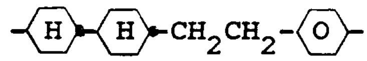 |
| (24) | Ethyl | 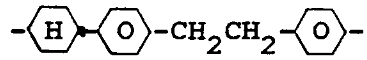 |
| (25) | n-Propyl | 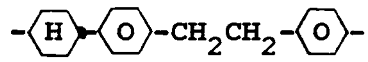 |
| (26) | n-Butyl | 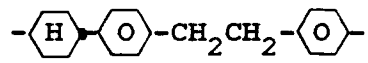 |
| (27) | n-Pentyl | 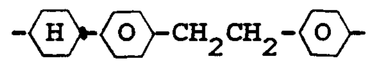 |
| (28) | Ethyl | 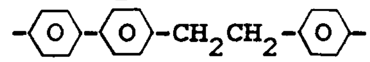 |
| (29) | n-Propyl | 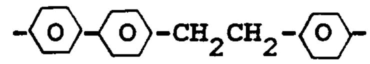 |
| (30) | n-Butyl | 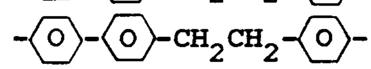 |
| (31) | n-Pentyl | 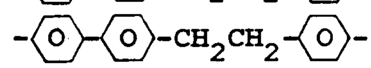 |
| (32) | Ethyl | 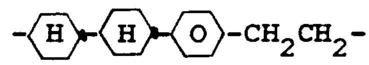 |
| (33) | n-Propyl | 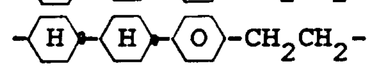 |
| (34) | n-Butyl | 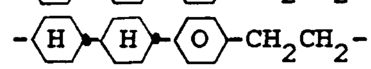 |
| (35) | n-Pentyl | 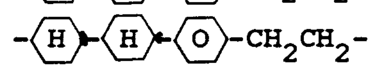 |
| (36) | Ethyl | 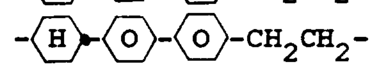 |
| (37) | n-Propyl | 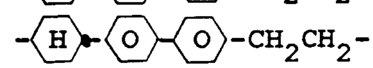 |
| (38) | n-Butyl | 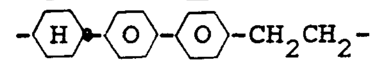 |
| (39) | n-Pentyl | 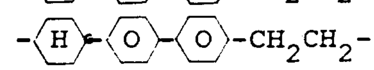 |

## Patentansprüche

1. Flüssigkristallines Medium für elektrooptische Anzeigeelemente mit mindestens zwei flüssigkristallinen Komponenten, dadurch gekennzeichnet, daß mindestens eine Komponente ein Difluorbenzonitril der

Formel I

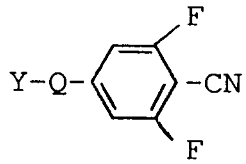

ist
worin

Y Alkyl, Alkenyl, Alkoxy, Oxaalkyl oder Alkenyloxy mit jeweils 1 bis 12 C-Atomen und

Q einen Rest ausgewählt aus der Gruppe bestehend aus

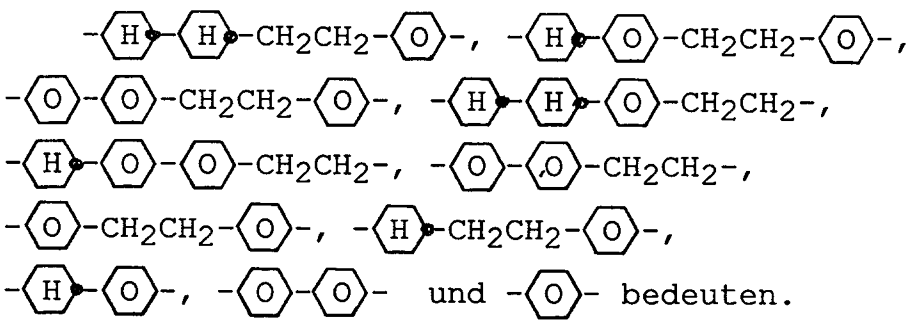

und bedeuten.

2. Elektrooptisches Anzeigeelemtent, dadurch gekennzeichnet, daß es ein Medium nach Anspruch 1 enthält.

3. Verwendung der Verbindungen der Formel I nach Anspruch 1 als Komponenten flüssigkristalliner Medien für elektrooptische Anzeigeelemente.

4. Difluorbenzonitrile der Formel I nach Anspruch 1 mit der Maßgabe, daß Q nicht

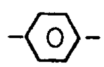

bedeutet.

## Claims

1. Liquid-crystalline medium *β* electrooptical display elements having at least two liquid-crystalline components, characterized in that at least one component is a difluorobenzonitrile of the formula I

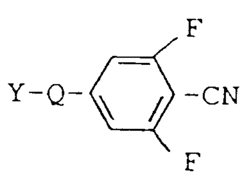

I

in which

Y is alkyl, alkenyl, alkoxy, oxaalkyl or alkenyloxy, in each case having from 1 to 12 carbon atoms, and

Q is a radical selected from the group comprising

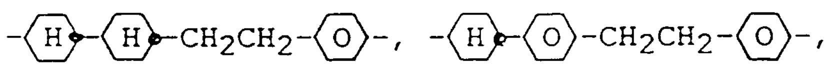
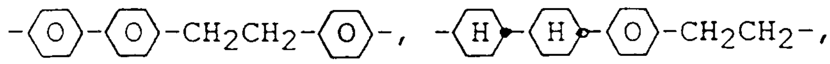
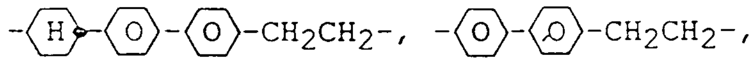
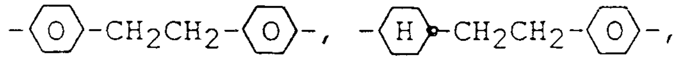
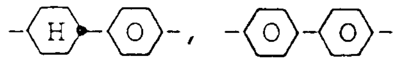 and 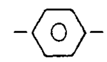

**2.** Electrooptical display element, characterized in that it contains a medium according to Claim 1.

**3.** Use of the compounds of the formula I according to Claim 1 as components of liquid-crystalline media for electrooptical display elements.

**4.** Difluorobenzonitriles at the formulat I according to Claim I, with the proviso that Q is not

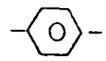 .

## Revendications

**1.** Milieu à cristaux liquides pour éléments d'affichage électro-optiques comprenant au moins deux composants à cristaux liquides, caractérisé en ce qu'au moins un composant est un difluorobenzonitrile de formule I

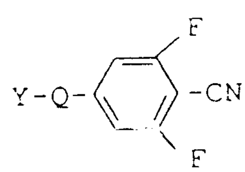

dans laquelle

| | |
|---|---|
| Y représente un | alkyle, alcényle, alcoxy, oxaalkyle ou alcényloxy avec à chaque fois de 1 à 12 atomes de carbone et |
| Q représente un | radical choisi dans le groupe constitué par |

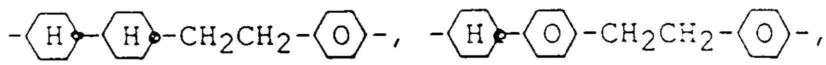
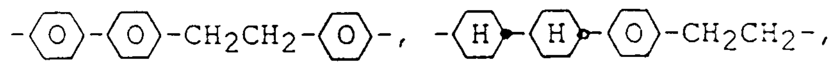
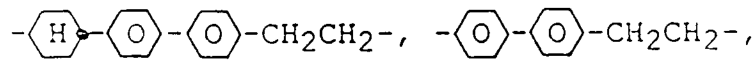
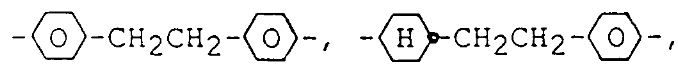
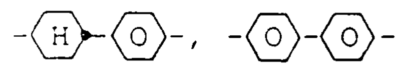 et 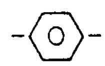 .

**2.** Elément d'affichage électro-optique, caractérisé en ce qu'il contient un milieu selon la revendication 1.

**3.** Application des composés de formule I selon la revendication 1 comme composants de milieux à cristaux liquides pour éléments d'affichage électro-optiques.

**4.** Difluorobenzonitriles de formule I selon la revendication 1, sous réserve que Q ne représente pas

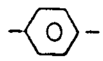 .